# EUROPÄISCHE PATENTSCHRIFT

(11) **EP 1 844 802 B1**
(45) Veröffentlichungstag und Bekanntmachung des Hinweises auf die Patenterteilung: **06.03.2013**
(21) Anmeldenummer: 07006760.8
(22) Anmeldetag: 31.03.2007
(51) Int. Cl.: A61M 3/02

(54) **Darmspülgerät mit einer Sicherheitseinrichtung in Form eines freien Auslaufs**
Intestine rinsing device with a safety device in the form of a free outlet
Appareil d'hydrothérapie du gros intestin doté d'un dispositif de sécurité en forme de purge libre

(30) Priorität: 12.04.2006 DE 202006006089 U
(43) Veröffentlichungstag der Anmeldung: 17.10.2007
(73) Patentinhaber: Eich, Helmut, 72805 Lichtenstein (DE)
(72) Erfinder: Eich, Helmut, 72805 Lichtenstein (DE)
(74) Vertreter: Ludewig, Rita

(56) Entgegenhaltungen:
- EP-B1- 0 329 599
- DE-A1- 3 842 396
- DE-A1- 19 907 594
- US-A- 5 199 604

## Beschreibung

Die Erfindung betrifft ein Darmspülgerät mit einer Sicherheitseinrichtung in Form eines freien Auslaufs zum Schutz des Trinkwassers, insbesondere zur Verhütung von Trinkwasserverunreinigungen durch Rücksaugen, Rückfließen oder Rückdrücken von verunreinigtem Wasser aus einem Darmspülgerät in die Trinkwasserleitung.

Das Betreiben von Geräten, die unmittelbar an die Trinkwasserleitung sowohl kalt- als auch warmwasserseitig angeschlossen werden ist gemäß DIN EN 1717 und DIN 1988 nur dann statthaft, wenn das Gerät eine eigene zwischengeschaltete gesonderte Sicherungseinrichtung, einen sogenannten freien Auslauf, aufweist. Das Prinzip des freien Auslaufs ist bekannt und wird in vielen Anwendungsbereichen in unterschiedlicher Form benutzt.

Es ist auch eine Sicherheitseinrichtung dieser Art für Darmspülgeräte bekannt, die unter dem Namen HABAMAT AQUACLEAN auf dem Markt angeboten wird. In einem separaten Gehäuse ist ein herkömmlicher Wasserboiler zum Erhitzen des zugeführten Leitungswassers und ein Wasserbehälter eingebaut. Der Wasserbehälter arbeitet nach dem Prinzip eines herkömmlichen Spülkastens, wie er zum Spülen von Toilettenbecken benutzt wird. Das Gehäuse ist nicht für eine gebrauchsbedingte Öffnung vorgesehen. Dieses Gerät wird als kompatibel mit allen auf dem Markt befindlichen Darmspülgeräten angeboten und erfüllt die vorgeschriebenen DIN- Normen zur Verhinderung der Verunreinigung der Trinkwasserleitungen.

Ungeachtet dessen, daß dieses Gerät, mit einem Gewicht von 32 kg und mit seinem Raumvolumen, daß ein Dreifaches des Raumbedarfs einiger auf dem Markt befindlicher Darmspülgeräte einnimmt, sehr schwer, unhandlich und teuer ist, weist es ein weiteres Problem auf. Der fest eingeschraubte Wasserbehälter, in Form eines Toilettenspülkastens, kann nicht regelmäßig gereinigt werden, ohne das Gerätegehäuse aufwendig demontieren zu müssen.

Bekanntermaßen setzen sich in Toilettenspülkästen, in denen nur Kaltwasser geführt wird, mit der Zeit erhebliche und unansehnliche Beläge ab. Diese Belagbildung wird bei ständiger Zufuhr von Warmwasser wie es bei Darmspülgeräten benötigt wird, noch verstärkt. Die Belagbildung schließt auf die Dauer auch die Bildung von Bakterien ein.

Da die, in dem bekannten Sicherheitssystem für Darmspülgeräte, eingebauten Spülkästen nie gereinigt werden, können sich in diesen nicht nur Beläge, sondern auch Bakterien ungehindert ansammeln und vermehren. Die Bakterien können zwar nicht in die Trinkwasserleitung gelangen, aber sie werden ungehindert und unkontrolliert mit dem warmen Wasser aus dem Spülkasten in den menschlichen Darm gepumpt, wo sie unbemerkt verheerende Wirkungen für die Gesundheit des Menschen hervorrufen können, deren Ursachen kein Mensch im Sicherheitssystem des Darmspülgerätes suchen wird.

Es war daher Aufgabe der Erfindung, eine Sicherheitseinrichtung, in Form eines freien Auslaufs, für Darmspülgeräte zu schaffen, welche die DIN- 1988 und DIN EU- 1717 für Darmspülgeräte und die einfachsten Hygienevorschriften, nämlich die Reinigung von Geräteteilen die für Eingriffe in den menschlichen Körper dienen, erfüllt, das neben dem erforderlichen Platzbedarf eines herkömmlichen Darmspülgerätes kein zusätzlicher Platz oder bei separatem Anschluß an kleinere Ausführungen von Darmspülgeräten nur wenig Platz benötigt, das leicht zu transportieren und zu bewegen ist und einen geringen Kostenaufwand erfordert.

Die Aufgabe wird durch ein Darmspülgerät mit einer Sicherheitseinrichtung in Form eines freien Auslaufs zum Schutz des Trinkwassers, insbesondere zur Verhütung von Trinkwasserverunreinigungen durch Rücksaugen, Rückfließen oder Rückdrücken von verunreinigtem Wasser aus einem Darmspülgerät in die Trinkwasserleitung, mit den kennzeichnenden Merkmalen des Anspruchs 1 gelöst.

Vorteilhaft ist der kleinräumig konstruktive Aufbau des Sicherheitssystems, das zu einem großen Teil im oder bedarfsweise am Gehäuse einer Vielzahl, auf dem Markt angebotener, Darmspülgeräte Platz findet und deshalb nachrüstbar ist.

Besonders hervorzuheben ist es, daß der Wasserbehälter durch seine optimale Größe und Handlichkeit und durch seine einfache und schnelle Verschließbarkeit, die durch einen Schnellverschluß am Verschlußdeckel gewährleistet ist, leicht und schnell austauschbar und regelmäßig, nach jeder Behandlung zu reinigen ist. Damit ist gewährleistet, daß die einfachsten Hygienevorschriften für Eingriffe in den menschlichen Körper, nämlich die regelmäßige Reinigung von Geräteteilen, erfüllt werden. Aus dem Wasserbehälter des freien Auslaufs kann kein mit Bakterien belastetes Wasser in den menschlichen Darm gelangen.

Vorteilhaft ist es weiterhin, daß der Verschlußdeckel mit den in ihm angeordneten Zuflußstutzen für das Warmwasser, dem Abflußstutzen mit Saugrohr zum Abpumpen des Warmwassers aus dem Wasserbehälter in den Wasserablaufschlauch zu einem Patienten und dem Entlüftungsstutzen sowie mit dem Schnellverschluß immer fest im Gehäuse des Darmspülgerätes oder in der bedarfsweise separat anschließbaren Sicherheitseinrichtung angeordnet ist und daß nur der Wasserbehälter gelöst, heraus- oder abzunehmen, zu reinigen, wieder einzusetzen und zu verschließen ist. Die positive Folge ist eine leichte und wenig zeitaufwendige Handhabung des Reinigungsvorgangs für die Bedienperson und der gesundheitserhaltende Schutz des Patienten.

Ein weiterer Vorteil der erfindungsgemäßen Lösung besteht darin, daß die Sicherheitseinrichtung durch ihren platzsparenden konstruktiven Aufbau und die Wahl bekannter leichgewichtiger Bauteile in einer Vielzahl von Darmspülgeräten nachrüstbar ist, ohne das Gehäuse vergrößern zu müssen. Die Anordnung des Wasserbehälters, in der Weise, daß der Wasserbehälter von außen abzunehmen, zu reinigen und wieder einzusetzen ist, ohne daß das Gehäuse des Darmspülgerätes geöffnet werden muß, ermöglicht eine weitere Verkürzung des Reinigungsprozesses.

Besonders hervorzuheben ist auch, gemäß Anspruch 2, das vergleichsweise geringe Gewicht der Sicherheitseinrichtung. Mit einem Gewicht von ca. 3000 g wird weder bei der Einbauvariante das Gewicht des Darmspülgerätes nennenswert erhöht, noch ist im Falle der Nutzung der Sicherheitseinrichtung als separater zu öffnender und zu verschließender Bausatz ein nennenswerter Platzbedarf erforderlich und eine leichte Handhabung gewährleistet. Die Nachrüstbarkeit in bereits in Benutzung befindlichen Darmspüleräten sowie die Ergänzung kleinvolumiger Darmspülgeräte mit einem ebenso kleinen und leichten, separaten Bausatz sichert darüber hinaus eine kostengünstige Weiterbenutzung der vorhandenen Geräte.

Schließlich ist die Einhaltung der DIN- 1988 und DIN EU- 1717 für Darmspülgeräte, gewährleistet, weil gemäß Anspruch 4, der vorgeschriebene Zwischenraum zwischen dem Wasserzulauf aus einer Warm- oder Kaltwasserleitung und dem Wasserspiegel im Wasserbehälter in Höhe von mindestens 20 mm einhaltbar und kontrollierbar ist. Außerdem ist sichergestellt, daß bei Erreichung die Marke von 20 mm, der Wasserzulauf zur Wasserleitung unterbrochen wird und ein Rückfluß, Rücksog oder Rückdruck von verunreinigtem Wasser in die Wasserleitung nicht möglich ist.

Die Erfindung soll nachstehend anhand von drei Ausführungsbeispielen näher beschrieben werden. Die Zeichnungen zeigen dabei in
- Fig. 1: die erfindungsgemäße Sicherheitseinrichtung in Form eines freien Auslaufs eingebaut in einem Darmspülgerät,
- Fig.2: eine Prinzipdarstellung des freien Auslaufs mit Magnetventil, Sensor, Elektronik, Verschlußdeckel, Behälter und Pumpe,
- Fig.3: die erfindungsgemäße Sicherheitseinrichtung in Form eines freien Auslaufs eingebaut in einem Darmspülgerät mit von außen auswechselbarem Behälter,
- Fig.4: die erfindungsgemäße Sicherheitseinrichtung in Form eines freien Auslaufs als separater zu öffnender und zu schließender Bausatz.

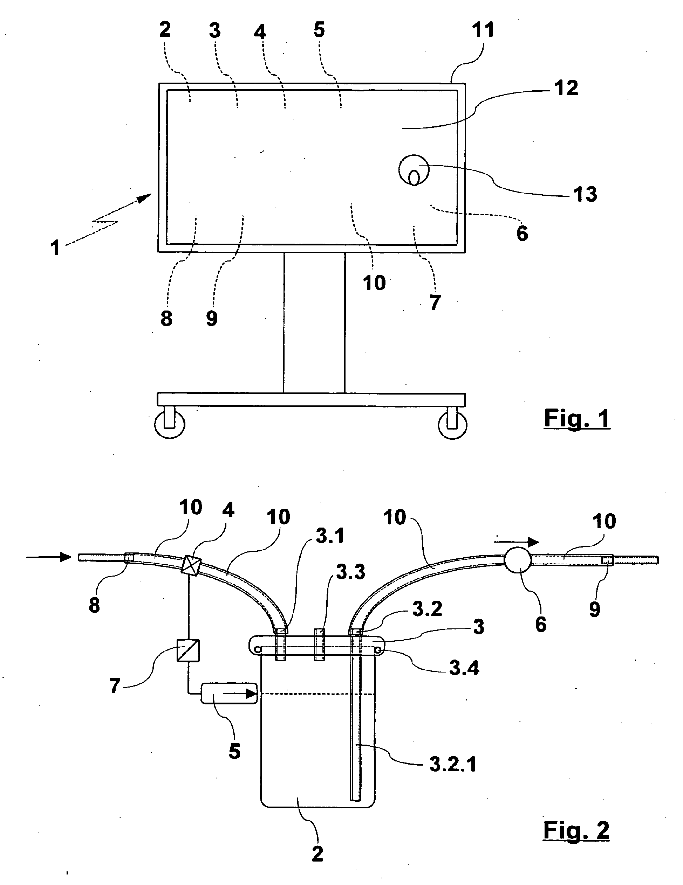

Im Ausführungsbeispiel, gemäß Fig.1, ist ein nicht näher dargestelltes Gehäuse 11 eines Darmspülgerätes zu sehen. In Fig.2 ist die prinzipielle funktionelle Verbindung der wesentlichen Bauteile des erfindungsgemäßen freien Auslaufs 1 dargestellt. Der in einem Darmspülgerät grundsätzlich vorhandene Anschlußsstutzen 8 für den Wasserzulauf und der Anschlußstutzen 9 für den Anschluß des Wasserzuflußschlauchs zum Patienten sind herkömmlich durch einen Wasserschlauch 10 miteinander verbunden. Dieser Wasserschlauch 10 wird durch einen erfindungsgemäß freien Auslauf 1 unterbrochen bzw. überbrückt. Am Wasserschlauch 10 ist ein Magnetventil 4 angeschlossen, das einerseits mit dem Zuflußstutzen 3.1 im Verschlußdeckel 3 und andererseits über eine Elektronik 7 mit einem Sensor 5 verbunden ist. Über den Zuflußstutzen 3.1 im Verschlußdeckel 3 fließt warmes Wasser aus der Warmwasserleitung in den Behälter 2 und bildet einen Wasserspiegel, der, gemäß DIN- EU 1717 und DIN 1988, einen Mindestabstand zum Zuflußsstutzen 3.1 in Höhe von 20 mm aufweist. Bei Erreichen dieser Abstandsmarke sendet der außerhalb des Behälters 2 angeordnete Sensor 5 ein Signal an das Magnetventil 4 und dieses unterbricht die Wasserzufuhr in den Behälter 2. Ein ebenfalls im Verschlußdeckel 3 angeordneter Abflußstutzen 3.2 weist ein Saugrohr 3.2.1 auf, das in das Wasser im Behälter 2 bis kurz über den Behälterboden hineinragt. Über eine, außerhalb des Wasserbehälters 2 am Abflußsstutzen 3.2 angeordnete, Pumpe 6 wird das Wasser aus dem Behälter 2 gepumpt und über den Anschlußstutzen 9 in den Wasserzulaufschlauch zum Patienten geleitet. Der außerdem im Verschlußdeckel 3 angeordnete Entlüftungsstutzen 3.3 entlüftet den Behälter 2. Der Verschlußdeckel 3, der Sensor 5 und die Pumpe 6 sind an der inneren Gehäusewand fest montiert. Der Behälter 2 wird nach dem Einführen des Saugrohrs 3.2.1 in den Behälter 2 an den Verschlußdeckel 3 herangeführt und mit einem herkömmlich bekannten Schnellverschluß 3.4 mit dem Verschlußdeckel 3 verbunden und dabei verschlossen. Danach wird die Gehäusetür 12 des Darmspülgerätes geschlossen. Nach Abschluß der Behandlung eines Patienten wird das Darmspülgerät vom Elektroanschluß getrennt, die Gehäusetür 12 durch zum Beispiel einen herkömmlichen abkippbaren Wirbelverschluß 13 geöffnet der Behälter 2 mit beispielsweise einem Wasseraufnahmevermögen von einem Liter mittels dem Schnellverschluß 3.4 vom Verschlußdeckel 3 getrennt, gereinigt und wie bereits beschrieben, nach Wiedereinführung des Saugrohrs 3.2.1. in den Behälter 2 zum Verschlußdeckel 2 geführt und mittels des Schnellverschlusses 3.4 durch leichte Drehung wieder verschlossen. Die Gehäusetür 12 wird geschlossen und die nächste Behandlung kann beginnen.

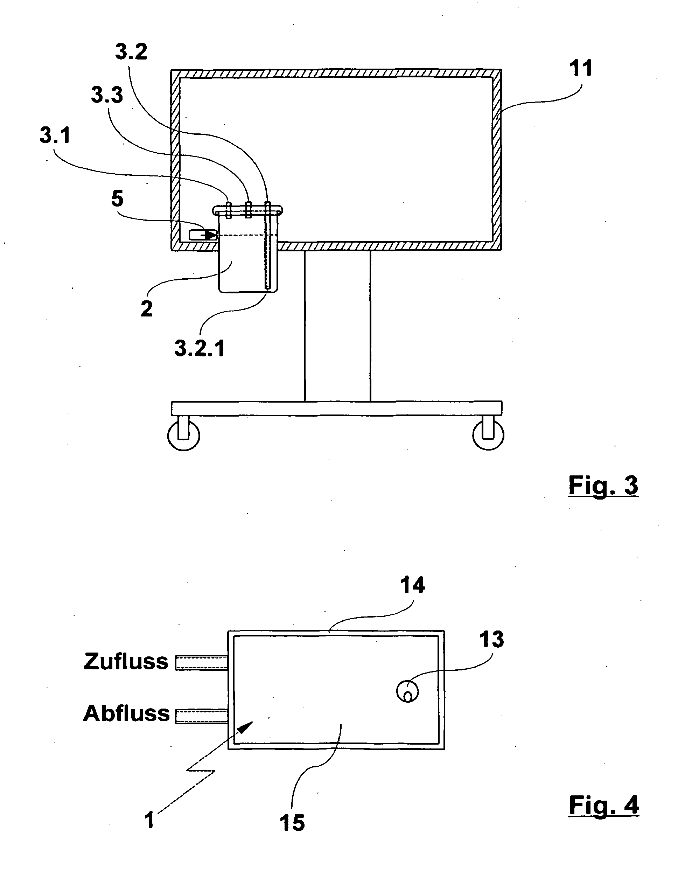

In einem zweiten Ausführungsbeispiel, gemäß Fig.3, ist der Verschlußdeckel 3 mit dem Zuflußstutzen 3.1, dem Abflußstutzen 3.2 mit dem Saugrohr 3.2.1 und dem Entlüftungsstutzen 3.4 derart an der Gehäusewand angeordnet, daß der Behälter 2 zu ca. 2/3 seiner Länge aus dem Gehäuse des Darmspülgerätes herausragt. Beim Reinigen des Behälters 2 muß dieser lediglich leicht gedreht, aus der Verankerung des Schnellverschlusses 3.4 des Verschlußdeckels 3 gelöst und nach unten herausgezogen werden. Nach der Reinigung wird das Saugrohr 3.2.1 in den Behälter 2 eingeführt der Behälter 2 in Richtung des Geräteinneren bewegt bis er am Verschlußdeckel 3 anstößt, leicht gedreht, über den Schnellverschluß 3.4 mit dem Verschlußdeckel 3 verbunden und gleichzeitig verschlossen. Ein Öffnen und Schließen des Gehäuses 11 entfällt.

Ein drittes Ausführungsbeispiel gemäß, Fig. 4, sieht ein separates Gehäuse 14 mit einer Gehäusetür 15 vor, in dem die Bauteile des erfindungsgemäßen freien Auslaufs 1 eingebaut sind. Dieser separate Bausatz kann mit einem herkömmlichen Darmspülgerät kleineren Vollumens gekoppelt werden. Durch Öffnen und Schließen der Gerätetür 15 kann der Behälter 2 ebenso ausgewechselt und gereinigt werden, wie das für das erste Ausführungsbeispiel, nach Fig.1, beschrieben wurde.

### Aufstellung der verwendeten Bezugszeichen

- 1: freier Auslauf,
- 2: Behälter,
- 3: Verschlußdeckel,
- 3.1: Zuflußstutzen,
- 3.2: Abflußstutzen,
- 3.2.1: Saugrohr,
- 3.3: Entlüftungsstutzen,
- 3.4: Schnellverschluß,
- 4: Magnetventil,
- 5: Sensor,
- 6: Pumpe,
- 7: Elektronik,
- 8: Wasserzulaufstutzen,
- 9: Wasserablaufstutzen,
- 10: Schlauch
- 11: Gehäuse,
- 12: Gehäusetür,
- 13: Verschluß,
- 14: separates Gehäuse,
- 15: Gehäusetür von 14

## Patentansprüche

1. Darmspülgerät mit einer Sicherheitseinrichtung in Form eines freien Auslaufs zum Schutz des Trinkwassers, unter anderem aufweisend ein Gehäuse (11) mit einer Gehäusetür (12) und einem Verschluß (13), in dem unter anderen ein Anschlußstutzen für den Wasserzulauf und ein Anschlußstutzen für den Anschluß eines Wasserablaufschlauchs zu einem Patienten angeordnet sind, die durch einen Wasserschlauch (10) miteinander verbunden sind, **dadurch gekennzeichnet,**
**daß** der Wasserschlauch (10) durch einen freien Auslauf (1) überbrückt ist, der einen Wasserbehälter (2), einen Verschlußdeckel (3) mit einem Zulaufstutzen (3.1) einem Abflußstutzen (3.2) und einem Entlüftungsstutzen (3.3), einen Schnellverschluß (3.4), ein Magnetventil (4), einen Sensor (5), eine Pumpe (6), eine Elektronik (7) zur Steuerung des Zusammenwirkens des Sensors (5) und des Magnetventils (4), einen Anschlußstutzen (8) für den Wasserzulauf und einen Anschlußstutzen (9) für den Wasserablauf aufweist und daß der Wasserbehälter (2) mit dem Verschlußdeckel (3) über den Schnellverschluß (3.4) miteinander verbunden ist, wobei nach Öffnung der Gehäusetür (12) mit dem Verschluß (13) der Wasserbehälter (2) des freien Auslaufs (1) über den Schnellverschluß (3.4) mit einer kurzen Drehbewegung zu entrasten, zu entnehmen sowie nach der Reinigung wieder einzusetzen und einzurasten ist und anschließend die Gehäusetür (12) mit dem Verschluß (13) wieder zu verschließen ist.

2. Darmspülgerät mit Sicherheitseinrichtung in Form eines freien Auslaufs (1), nach Anspruch 1, **dadurch gekennzeichnet, daß**, in einer zweiten Variante, der Verschlußdeckel (2) mit dem Zulaufstutzen (3.1), dem Ablaufstutzen (3.2) mit dem Saugrohr (3.2.1), dem Entlüftungsstutzen (3.3) und dem Schnellverschluß (3.4) derart an der Wand des Gehäuses (11) des Darmspülgerätes angeordnet ist, daß der Wasserbehälter (2) aus dem Gehäuse (11) herausragt, wodurch der Wasserbehälter (2) austauschbar zu reinigen und wieder einzusetzen ist, ohne das Darmspülgerät öffnen zu müssen.

3. Darmspülgerät mit Sicherheitseinrichtung in Form eines freien Auslaufs (1), nach Anspruch 1, **dadurch gekennzeichnet, daß** der freie Auslauf (1) mit allen seinen Bestandteilen in einem Darmspülgerät nachrüstbar ist und bei Darmspülgeräten mit kleinem Gehäusevolumen als separater zu öffnender und zu verschließender Bausatz mit einem Gesamtgewicht von etwa 3000 g zu koppeln ist.

4. Darmspülgerät mit Sicherheitseinrichtung in Form eines freien Auslaufs (1), nach Anspruch 1, **dadurch gekennzeichnet, daß** das Wasser aus der Trinkwasserleitung über den Anschlußstutzen (8) für den Wasserzulauf, den Wasserschlauch (10) und den Zulaufstutzen (3.1) in den Wasserbehälter (2) läuft und von der Pumpe (6) über das Saugrohr (3.2.1) und den Ablaufstutzen (3.2) aus dem Wasserbehälter (2) über den Wasserschlauch (10) und den Anschlußstutzen (9) in den Wasserzulaufschlauch zum Patienten zu pumpen ist.

5. Darmspülgerät mit Sicherheitseinrichtung in Form eines freien Auslaufs, nach Anspruch 1, **dadurch gekennzeichnet, daß** zwischen dem Zulaufstutzen (3.1) im Wasserbehälter (2) und einem, der DIN- Norm gemäß, definierten Wasserspiegel, ein geforderter Zwischenraum von mindestens 20mm von dem Sensor (5) zu überwachen ist, wobei bei Erreichen der 20mm- Marke der Sensor (5) über die Elektronik (7) dem Magnetventil (4) ein Signal sendet, wodurch die Verbindung zur Trinkwasserleitung zu unterbrechen ist.

6. Darmspülgerät mit Sicherheitseinrichtung in Form eines freien Auslaufs, nach Anspruch 1, **dadurch gekennzeichnet, daß** der Wasserbehälter (2) aus einem, im medizinischen Bereich, zugelassenen Material besteht und beim Austausch und der Reinigung des Wasserbehälters (2) für die Bedienperson keine körperliche Belastung und kein die Kosten erhöhender Zeitaufwand notwendig ist.

## Claims

1. Colonic irrigation machine with a safety device in the form of a free outlet for protecting drinking water, inter alia having a housing (11) with a housing door (12) and a locking device (13) in which, inter alia, a connecting piece for water intake and a connecting piece for connecting a water outlet hose to a patient are arranged, which are connected to each other by means of a water hose (10), **characterised in that**
the water hose (10) is bridged by means of a free outlet (1) which has a water container (2), a cover lid (3) with an inlet nozzle (3.1), an outlet nozzle (3.2) and a ventilation nozzle (3.3), a quick-release fastener (3.4), a solenoid valve (4), a sensor (5), a pump (6), an electronic system (7) for controlling the interaction of the sensor (5) and the solenoid valve (4), a connecting piece (8) for water intake and a connecting piece (9) for water outlet, and that the water container (2) is connected to the cover lid (3) via the quick-release fastener (3.4), wherein after opening the housing door (12) with the locking device (13), the water container (2) of the free outlet (1) can be released and removed with a short rotary movement via the quick-release fastener (3.4), and then reinserted and engaged after cleaning, the housing door (12) subsequently being able to be closed again with the locking device (13).

2. Colonic irrigation machine with a safety device in the form of a free outlet (1), according to claim 1, **characterised in that**, in a second variation, the cover lid (3) is arranged with the inlet nozzle (3.1), the outlet nozzle (3.2), the suction pipe (3.2.1), the ventilation nozzle (3.3) and the quick-release fastener (3.4) on the wall of the housing (11) of the colonic irrigation device in such a way that the water container (2) protrudes from the housing (11), whereby the water container (2) can be removed for cleaning purposes and then re-attached without having to open the colonic irrigation machine.

3. Colonic irrigation machine with a safety device in the form of a free outlet (1), according to claim 1, **characterised in that** the free outlet (1) together with all its components can be retrofitted in a colonic irrigation machine and in the case of colonic irrigation machines with a small housing volume, can be connected as a separate kit with a total weight of approximately 3000 g, which can be opened and closed.

4. Colonic irrigation machine with a safety device in the form of a free outlet (1), according to claim 1, **characterised in that**, for the intake of water, the water flows from the drinking water supply via the connecting piece (8), the water hose (10) and the inlet nozzle (3.1) into the water container (2), and is pumped by the pump (6) via the suction pipe (3.2.1) and the outlet nozzle (3.2) out of the water container (2) via the water hose (10) and the connecting piece (9) into the water inlet hose to the patient.

5. Colonic irrigation machine with a safety device in the form of a free outlet, according to claim 1, **characterised in that**, between the inlet nozzle (3.1) in the water container (2) and a water level defined according to the DIN standard, a stipulated gap of at least 20 mm should be monitored by the sensor (5), wherein when the 20 mm point is reached, the sensor (5) sends a signal via the electronic system (7) to the solenoid valve (4), whereby the connection to the drinking water supply is cut off.

6. Colonic irrigation machine with a safety device in the form of a free outlet, according to claim 1, **characterised in that** the water container (2) consists of a material approved for medical applications, and when replacing and cleaning the water container (2), neither physical stress on the part of the operator is necessary, nor expenditure of time which increases costs.

## Revendications

1. Appareil d'irrigation du côlon, comprenant un dispositif de sécurité sous forme d'une sortie libre pour la protection de l'eau potable, comprenant entre autres un boîtier (11) ayant une porte de boîtier (12) et une fermeture (13) et à l'intérieur duquel sont disposées, entre autres, une tubulure de raccordement pour l'amenée d'eau et une tubulure de raccordement pour raccorder un tuyau flexible d'évacuation d'eau vers un patient, qui sont reliées entre elles par un tuyau flexible d'eau (10), **caractérisé par le fait que**
le tuyau flexible d'eau (10) est ponté par une sortie libre (1) qui comprend un réservoir d'eau (2), un couvercle de fermeture (3) ayant une tubulure d'admission (3.1), une tubulure d'évacuation (3.2) et un raccord de purge (3.3), une fermeture rapide (3.4), une électrovanne (4), un capteur (5), une pompe (6), une électronique (7) de commande de la coopération du capteur (5) et de l'électrovanne (4), une tubulure de raccordement (8) pour l'amenée d'eau et une tubulure de raccordement (9) pour l'évacuation d'eau, et **par le fait que** ledit réservoir d'eau (2) est relié au couvercle de fermeture (3) par le biais de la fermeture rapide (3.4), après avoir ouvert la porte de boîtier (12) au moyen de ladite fermeture (13), ledit réservoir d'eau (2) de ladite sortie libre (1) étant désenclenché par ladite fermeture rapide (3.4) avec un court mouvement de rotation, étant retiré et, après le nettoyage, remis en place et à nouveau encliqueté, et, ensuite, la porte de boîtier (12) étant refermée au moyen de ladite fermeture (13).

2. Appareil d'irrigation du côlon, comprenant un dispositif de sécurité sous forme d'une sortie libre (1), selon la revendication 1, **caractérisé par le fait que**, dans une deuxième variante, ledit couvercle de fermeture (3) avec ladite tubulure d'admission (3.1), avec ladite tubulure d'évacuation (3.2) comprenant le tuyau d'aspiration (3.2.1), avec ledit raccord de purge (3.3) et avec ladite fermeture rapide (3.4) est disposé sur la paroi du boîtier (11) de l'appareil d'irrigation du côlon de telle sorte que ledit réservoir d'eau (2) fait saillie hors du boîtier (11), le réservoir d'eau (2) pouvant ainsi être nettoyé de manière échangeable et être remis en place sans que l'appareil d'irrigation du côlon doive être ouvert.

3. Appareil d'irrigation du côlon, comprenant un dispositif de sécurité sous forme d'une sortie libre (1), selon la revendication 1, **caractérisé par le fait que** ladite sortie libre (1) avec l'ensemble de ses composants peut être montée ultérieurement à l'intérieur d'un appareil d'irrigation du côlon et, dans le cas d'appareils d'irrigation du côlon présentant un petit volume de boîtier, être couplée en tant que kit séparé à ouvrir et à fermer, ayant un poids total de 3000 g à peu près.

4. Appareil d'irrigation du côlon, comprenant un dispositif de sécurité sous forme d'une sortie libre (1), selon la revendication 1, **caractérisé par le fait que** l'eau en provenance de la conduite d'eau potable atteint le réservoir d'eau (2) en passant par ladite tubulure de raccordement (8) pour l'amenée d'eau, par le tuyau flexible d'eau (10) et par ladite tubulure d'admission (3.1), et est pompée par ladite pompe (6) via le tuyau d'aspiration (3.2.1) et la tubulure d'évacuation (3.2) hors du réservoir d'eau (2) pour atteindre le tuyau flexible d'amenée d'eau vers le patient en passant par le tuyau flexible d'eau (10) et la tubulure de raccordement (9).

5. Appareil d'irrigation du côlon, comprenant un dispositif de sécurité sous forme d'une sortie libre, selon la revendication 1, **caractérisé par le fait que**, entre ladite tubulure d'admission (3.1) dans ledit réservoir d'eau (2) et un niveau d'eau défini selon la norme DIN, un espace requis d'au moins 20 mm est surveillé par ledit capteur (5), ledit capteur (5) envoyant un signal via ladite électronique (7) à ladite électrovanne (4) lorsque ledit point de repère de 20 mm est atteint, ce par quoi la liaison à la conduite d'eau potable est interrompue.

6. Appareil d'irrigation du côlon, comprenant un dispositif de sécurité sous forme d'une sortie libre, selon la revendication 1, **caractérisé par le fait que** ledit réservoir d'eau (2) est réalisé dans une matière admise dans le domaine médical et que l'échange et le nettoyage du réservoir d'eau (2) ne causent aucun effort physique pour l'opérateur et n'exigent pas un temps important qui fait augmenter les coûts.
